# EUROPEAN PATENT SPECIFICATION

(11) **EP 4 149 337 B1**
(45) Date of publication and mention of the grant of the patent: **02.10.2024**
(21) Application number: 21722617.4
(22) Date of filing: 06.04.2021
(51) Int. Cl.: A47L 11/24, A47L 11/28, A47L 11/30, A47L 11/38

(54) **FLUID DISPENSING SYSTEM AND METHOD**
FLÜSSIGKEITSAUFTRAGSSYSTEM UND VERFAHREN
SYSTÈME DE DISPERSION DE FLUIDE ET MÉTHODE

(30) Priority: 11.05.2020 US 202063023039 P
(43) Date of publication of application: 22.03.2023
(73) Proprietor: Tennant Company, Eden Prairie, MN 55344 (US)
(72) Inventor: MESSER, Daniel, Minneapolis, MN 55410 (US); NORTRUP, Eric S., Minneapolis, MN 55422 (US); PYLKKI, Russell J., Saint Paul, MN 55116 (US); CARLSON, Brett, Rogers, MN 55374 (US)
(74) Representative: Reddie & Grose LLP
(86) International application number: PCT/US2021/025840
(87) International publication number: WO 2021/230996

(56) References cited:
- EP-A1- 0 286 328
- EP-A2- 3 508 105
- DE-U1- 202011 003 518
- DE-U1- 9 307 652

## Description

### CROSS-REFERENCE TO RELATED APPLICATION

### TECHNICAL FIELD

This disclosure relates to dispensing fluid and, more particularly, to dispensing fluid on a surface to treat the surface.

### BACKGROUND

Sanitization of large public spaces is difficult, resource, and time consuming. Manual sanitization, using wipes for example, is also unreliable on complex surface areas with many hard to access areas, such as a retail rack with many products on it, or a hospital environment with medical devices mounted on the walls.

Dispensing of sanitizing fluids using portable foggers has been used extensively over the years and has been proven efficient as the fog will deposit disinfectant droplets on all areas where a microorganism would have an opportunity to be located. However, it is time-consuming and resource intensive.

DE 9307652 U1 describes a cleaning system used to clean sieve-like trays. A mobile cleaning trolley is provided in such a way that the cleaning trolley has a mobile substructure on which at least one spatially movable nozzle arrangement for dispensing water and/or cleaning agent and/or disinfectant are provided. The mobile cleaning trolley is equipped chains and resembles a ramp vehicle. EP-A-0286328 discloses a surface maintenance machine according to the preamble of claim 1.

### SUMMARY

The features of the claimed invention are provided in the independent claims, to which reference should now be made. Additional, optional features are provided in the dependent claims.

The details of one or more examples are set forth in the accompanying drawings and the description below. Other features, objects, and advantages will be apparent from the description and drawings, and from the embodiments.

### BRIEF DESCRIPTION OF DRAWINGS

FIG. 1A is a perspective view of a mobile surface maintenance machine according to an example of the present disclosure.
FIG. 1B is a sectional perspective view of the mobile surface maintenance machine of FIG. 1A taken along a longitudinal plane through the mobile surface maintenance machine.
FIG. 1C is a top-down view of the surface maintenance machine of FIG. 1A and FIG. 1B including a maintenance operation zone.
FIG. 2 is a schematic view of an example system including a fluid dispensing system connected to a surface maintenance machine.
FIG. 3 is a schematic view of an alternate embodiment of a fluid dispensing system with an electrostatic module.
FIG. 4A and FIG. 4B are front elevation views of an example system including a fluid dispensing system connected to a surface maintenance machine.
FIG. 5A is a top-down view of an example system including two groups of nozzles on a left and right side of a surface maintenance machine.
FIG. 5B is a top-down view of an example system including three groups of nozzles systems on a left, right, and back side of a surface maintenance machine.
FIG. 5C is a top-down view of an example system including one group of nozzles on a single side of a surface maintenance machine.

### DETAILED DESCRIPTION

The following detailed description is exemplary in nature and is not intended to limit the scope, applicability, or configuration of the invention in any way. Rather, the following description provides some practical illustrations for implementing examples of the present invention. Examples of constructions, materials, dimensions, and manufacturing processes are provided for selected elements, and all other elements employ that which is known to those of ordinary skill in the field of the invention. Those skilled in the art will recognize that many of the noted examples have a variety of suitable alternatives.

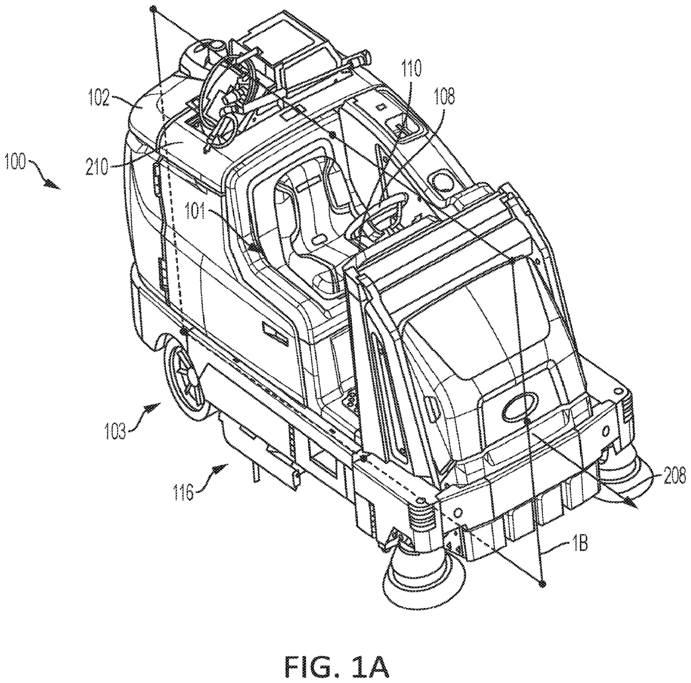

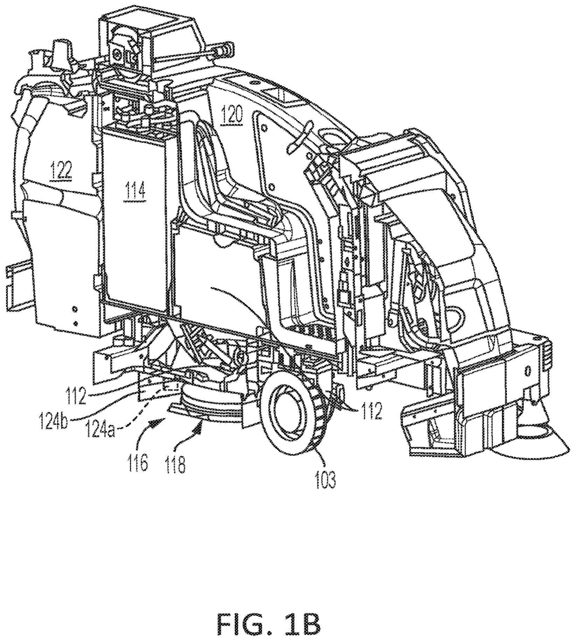

FIG. 1A to FIG. 1B are perspective views of an example surface maintenance machine 100 according to an aspect of the invention. FIG. 1A is a perspective view of the surface maintenance machine. FIG. 1B is a perspective sectional view taken through the longitudinal plane 1B in FIG. 1A. Referring to the illustrated embodiments shown in FIG. 1A to FIG. 1B, the surface maintenance machine 100 is a ride-on machine. During use, an operator may ride the surface maintenance machine 100 in a seated position and sit in the seat in the operator cab 101. Alternatively, the machine can be a walk-behind or a tow-behind machine.

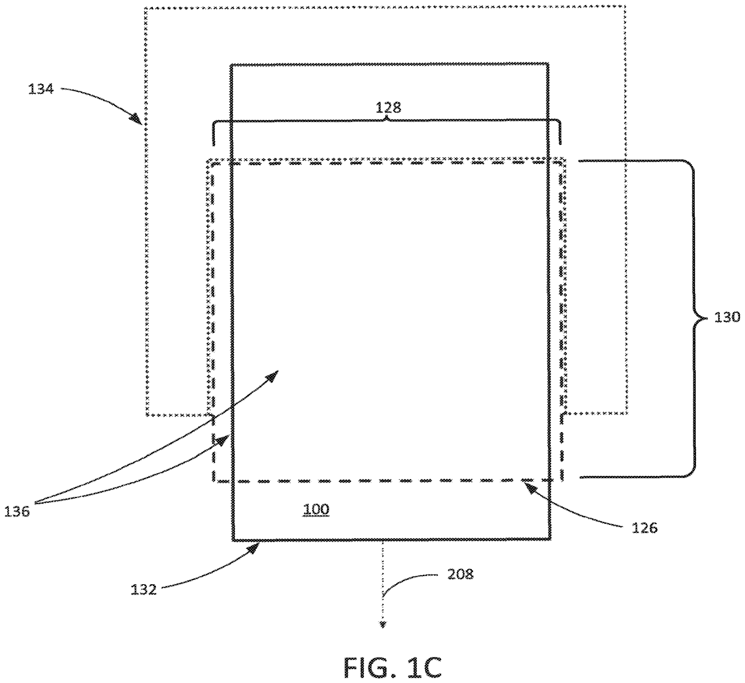

The surface maintenance machine can perform maintenance tasks such as sweeping, scrubbing, polishing (burnishing) a surface. The surface can be a floor surface, pavement, road surface and the like. In the example of FIG. 1C, a portion of the surface can be defined as a maintenance operation zone 126. In some examples, the maintenance operation zone 126 includes an area on which and only within which surface maintenance equipment 136 of a surface maintenance machine is performing a surface maintenance operation at a point in time or at a particular location of the surface maintenance machine. Alternatively, the surface maintenance machine performs a surface maintenance operation on an area which includes the maintenance operation zone 126 at a point in time or at a particular location of the machine. The maintenance operation zone can include a lateral extent 128 and a longitudinal extent 130. The maintenance operation zone typically includes a surface area on which a maintenance head assembly performs a surface maintenance operation as part of the surface maintenance equipment 136. Additional surface maintenance equipment 136 may be performing the surface maintenance operation within the surface maintenance zone. The maintenance operation zone in FIG. 1C is an area in which a cleaning fluid is dispensed thereon, a maintenance head assembly 116 cleans the area using a scrubber or other surface maintenance tool, and a vacuum/vacuum squeegee removes any fluid from the maintenance operation zone 126. Accordingly, the surface maintenance equipment 136 in such a design also includes a vacuum / vacuum squeegee assembly and any dispensing equipment (e.g., cleaning fluid nozzles typically located forward of the maintenance head assembly) that dispenses the cleaning fluid. In FIG. 1C, the lateral extent 128 of the maintenance operation zone 126 extends further than the lateral extent of the body 132 of the surface maintenance machine 100, which is common when the maintenance equipment includes a vacuum squeegee that extends slightly wider than the body 132 of the surface maintenance machine. In examples that exclude a vacuum squeegee (e.g., a sweeper surface maintenance machine or a polisher surface maintenance machine), the maintenance operation zone 126 may be narrower than the body 132 such that the lateral extent of the maintenance operation zone 126 is less than the lateral extent of the body 132. In either example, a surface maintenance operation is only performed within the lateral extent of the maintenance operation zone 126 and not outside of such zone.

The longitudinal extent 130 of the maintenance operation zone 126 may not extend along the entire length of the body 132 of the surface maintenance machine 100. For instance, as shown in FIG. 1C, the body 132 of the surface maintenance machine extends rearward of the maintenance operation zone 126. In this example, vacuum squeegee is located in the rear portion of the maintenance operation zone 126, such that, a point in time or at a location of the surface maintenance machine, the surface rearward of the vacuum squeegee is not receiving a surface maintenance operation. To the extent that the surface maintenance machine includes a vacuum and not a vacuum squeegee, the longitudinal extent of the maintenance operation zone 126 may be very similar to that shown in FIG. 1C. Typically, the floor maintenance operation does not extend rearward of the vacuum, such that part of the body of the floor maintenance machine may extend rearward of the maintenance operation zone 126.

In the example of FIG. 1C, a treatment fluid area 134 is also included. The treatment fluid area 134 extends beyond (e.g., rightward, leftward, and/or rearward) the body 132 of the surface maintenance machine 100 and beyond (e.g., rightward, leftward, and/or rearward) of the maintenance operation zone 126. The treatment fluid area 134 is typically outside of the maintenance operation zone 126, because any treatment fluid dispensed into the maintenance operation zone 126 will likely be part of the maintenance operation (e.g., acted on by one or more of the surface maintenance equipment 136 devices) and not part of a treatment fluid operation like that would occur in the treatment fluid area 134. In some examples, the forwardmost area of the treatment fluid area 134 is rearward of the maintenance operation zone 126 as defined by the forward direction of travel 208. In some examples, the forwardmost area of the maintenance operation zone 126 is rearward of the forwardmost area covered by the body 132 of the surface maintenance machine 100.

Examples of the surface maintenance machine 100 include components that are supported on a mobile body 102. The mobile body 102 comprises a frame supported on wheels 103 for travel over a surface, on which a surface maintenance operation is to be performed. The mobile body 102 may include operator controls and a steering control such as a steering wheel 108 such that an operator can turn the steering wheel 108 and control the speed of the surface maintenance machine 100 without having to remove the operator's hands from the steering wheel 108 using means well-known in the art. Continuing with the illustrated embodiment of FIG. 1A to FIG. 1B, advantageously, controls for steering, propelling, and controlling various operations of the surface maintenance machine 100 can be provided on an operator console 110.

In an aspect of the design, the surface maintenance machine 100 may be powered by batteries 114. The batteries 114 can be proximate the rear of the surface maintenance machine 100, or it may instead be located elsewhere, such as within the interior of the surface maintenance machine 100, supported within a frame, and/or proximate the front of the surface maintenance machine 100. Alternatively, the surface maintenance machine 100 can be powered by an external electrical source (e.g., a power generator) via an electrical outlet 276 or a fuel cell.

The surface maintenance machine 100 includes one or electric motors 112 that are supported on the mobile body 102 and may be located within the interior of the surface maintenance machine 100. One or more electric motors 112 receive power from batteries 114. Electric motors 112 supply torque to the surface maintenance machine, including the torque to rotate the wheels 103 in order to propel the surface maintenance machine 100 in a selected direction such as the forward direction of travel. Alternatively, the surface maintenance machine 100 includes one or more engines that may generate electrical power for use by the surface maintenance machine and/or that directly drive portions of the surface maintenance machine.

The surface maintenance machine 100 includes a maintenance head assembly 116 (sometimes the assembly is referred to as a maintenance head). The maintenance head assembly 116 houses one or more motor-driven surface maintenance tools 118 such as scrub brushes, sweeping brushes, and polishing, stripping or burnishing pads, and tools for extracting (e.g., dry or wet vacuum tools). For example, the maintenance head assembly 116 is a cleaning head comprising one or more cleaning tools (e.g., sweeping or scrubbing brushes) as motor-driven surface maintenance tools 118. Alternatively, the maintenance head assembly 116 is a treatment head comprising one or more cleaning tools (e.g., polishing, stripping or buffing pads). Many different types of motor-driven surface maintenance tools are used to perform one or more maintenance operations on the surface or on a portion of the surface, such as the maintenance operation zone 126 in FIG. 1C. The maintenance operation can be a dry operation or a wet operation. In a wet operation, fluid, such as a treatment fluid (e.g., a cleaning fluid) from a fluid reservoir 120, is supplied to or proximate to the maintenance head assembly 116 where it may be sprayed onto the underlying floor surface. Such maintenance tools include sweeping, scrubbing brushes, wet scrubbing pads, polishing/burnishing and/or buffing pads. Additionally, one or more side brushes for performing sweeping, dry or wet vacuuming, extracting, scrubbing or other operations can be provided. The maintenance head assembly 116 can extend toward a surface on which a maintenance operation is to be performed. For example, the maintenance head assembly 116 can be attached to the base of the surface maintenance machine 100 such that the head can be lowered to an operating position and raised to a traveling position. The maintenance head assembly 116 can be connected to the surface maintenance machine 100 using any known mechanism, such as a suspension and lift mechanism. The torque for the maintenance head may be provided by the one or more electric motors 112. In an aspect of the invention, different ones of the one or more electric motors provide the torque to propel the machine and provide the torque to actuate components of the maintenance head assembly 116, such as the one or more motor-driven surface maintenance tools.

Referring to FIG. 1A to FIG. 1C, in some embodiments, the interior of the surface maintenance machine 100 can include a vacuum system for removal of substances, such as debris or liquid, from the surface. The vacuum system can include a vacuum inlet 124a connected to a vacuum source which allows debris or fluid to be sucked up from the vacuum inlet 124a by the vacuum source. In some examples, the vacuum inlet 124a is part of a vacuum squeegee 124b which can remove liquid from the surface or from a portion of the surface such as along the lateral extent 128 of the maintenance operation zone 126. The vacuum squeegee 124b can be mounted such that it extends from a lower reward portion of the surface maintenance machine 100. In some examples, the vacuum inlet 124a is a sweeper vacuum, which can remove solid debris from the surface or from a portion of the surface such as along the lateral extent of the maintenance operation zone. In some of such embodiments, the interior of the surface maintenance machine 100 can include a fluid reservoir 120 and a fluid recovery tank 122. The fluid reservoir 120 can include a fluid source such as a treatment fluid that can be applied to the surface during treating operations. In advantageous embodiments, the treatment fluid can be cleaning fluid, a sanitizing fluid, or any fluid, such as water. In some embodiments, the reservoir contains a diluent, such as water, which can be added to a concentrated solution of the treatment fluid. The fluid recovery tank 122 holds recovered fluid that has been applied to the surface and soiled. The interior of the surface maintenance machine 100 can include passageways for passage of debris and dirty liquid. In some such cases, the vacuum system can be fluidly coupled to the fluid recovery tank 122 for drawing dirt, debris or soiled liquid from the surface. Fluid, for example, clean liquid, which may be mixed with a detergent, can be dispensed from the fluid reservoir 120 to the floor beneath surface maintenance machine 100, in proximity to the scrubbing brushes, and soiled scrubbing fluid is drawn by the squeegee centrally, after which it is suctioned via a recovery hose into the fluid recovery tank 122. In some examples, the vacuum squeegee can remove soiled fluid indirectly from the floor. One such example is a vacuum squeegee mounted to the maintenance head assembly such that it removes soiled fluid, which has been in contact with the floor, directly from the scrubbing brushes. In other examples, more than one vacuum squeegee is used, such as a vacuum squeegee for removing fluid directly from the brushes along with a vacuum squeegee extending from a lower reward portion of the surface maintenance machine. Surface maintenance machine 100 can also include a feedback control system to operate these and other elements of surface maintenance machine 100, according to apparatus and methods which are known to those skilled in the art.

In alternative embodiments, the surface maintenance machine 100 may be combination sweeper and scrubber machines. In such embodiments, in addition to the elements describe above, the surface maintenance machine 100 may either be an air sweeper-scrubber or a mechanical sweeper-scrubber. Such a surface maintenance machine 100 can also include sweeping brushes (e.g., rotary broom) extending toward a surface (e.g., from the underside of the surface maintenance machine 100), with the sweeping brushes designed to direct dirt and debris into a hopper. In the cases of an air sweeper-scrubber, the surface maintenance machine 100 can also include a vacuum system for suctioning dirt and debris from the surface. In still other embodiments, the surface maintenance machine 100 may be a sweeper. In such embodiments, the surface maintenance machine 100 may include the elements as described above for a sweeper and scrubber surface maintenance machine 100, but would not include the scrubbing elements such as scrubbers, squeegees and fluid storage tanks (for detergent, recovered fluid and clean liquid).

In further alternative embodiments, the surface maintenance machine 100 may be a sweeper without scrubber functionality, such as an air sweeper-scrubber or a mechanical sweeper. In such embodiments, the surface maintenance machine 100 may include the elements as described above for a sweeper and scrubber surface maintenance machine 100, but would not include the scrubbing elements such as scrubbers, squeegees and fluid storage tanks (for detergent, recovered fluid and clean liquid). The maintenance head of such a sweeper may comprise sweeping brushes (e.g., rotary broom) extending toward a surface (e.g., from the underside of the surface maintenance machine 100), with the sweeping brushes designed to direct substances, such as dirt and debris into a hopper. In the cases of an air sweeper-scrubber, the surface maintenance machine 100 can also include a vacuum system for suctioning substances, including dirt and debris from the surface.

In some examples, the surface maintenance machine 100 can include a primary vacuum source and a secondary vacuum source. The primary vacuum source can be used to provide a vacuum force for a vacuum squeegee while the secondary vacuum source can be used to provide a vacuum force for a sweeper vacuum. In some examples, the primary vacuum source and/or the secondary vacuum source can have an exhaust to exhaust air made by the primary and/or secondary vacuum source. The exhaust air can be used to provide air pressure.

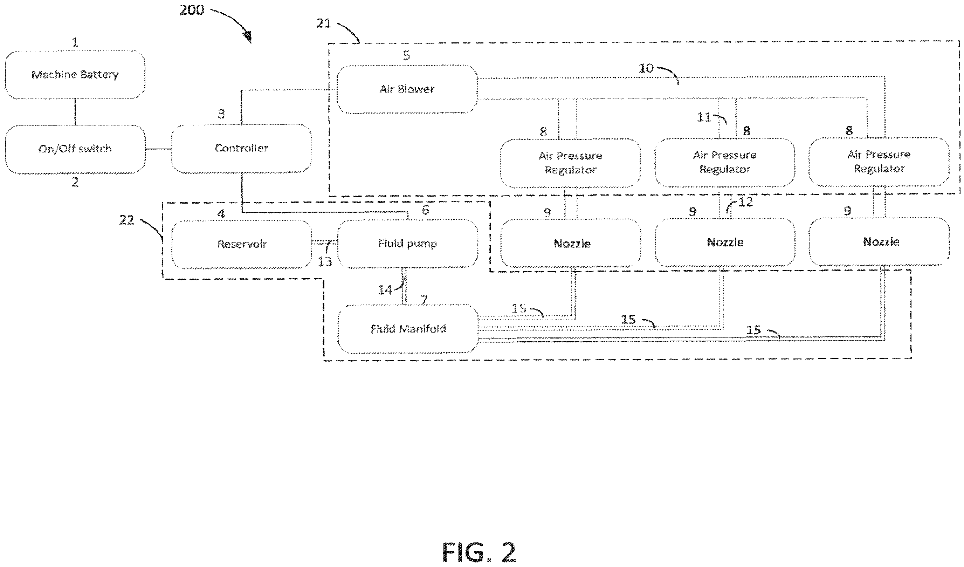

Moving to FIG. 2, FIG. 2 is a schematic view of an example fluid dispensing system 200 coupled to a surface maintenance machine (e.g., the surface maintenance machine 100 of FIG 1A). The fluid dispensing system 200 is powered by the surface maintenance machine's batteries 1 and is operated by a power switch 2 which provides current to the fluid dispensing system when turned on. In some examples, a battery, separate from the machine battery 1, provides power to the fluid dispensing system 200. In the example of FIG. 2, power switch 2 is connected to a controller 3 which can control certain aspects of the fluid dispensing system 200 as is further described herein.

The fluid dispensing system of FIG. 2 dispenses a fluid. In some examples, the fluid is a treatment fluid. In some examples, the treatment fluid is a sanitizing solution or concentrate solution. In such examples, the treatment fluid will treat the surface on which it is dispensed in a manner that may sanitize or disinfect the surface. It will be appreciated that other fluids and solutions can be used and are contemplated. In some examples, the treatment fluid is the same solution used by the surface maintenance machine to clean a floor surface. In other examples, the treatment fluid used by the fluid dispensing system is different from the treatment fluid used by the surface maintenance machine. In some examples, the fluid dispensing system of FIG. 2 can include ultra-low volume (ULV) techniques for dispensing the fluid in a fog, mist, or spray.

In the example of FIG. 2, an air delivery system 21 includes an air blower 5, primary hose 10, secondary hoses 11, and air pressure regulators 8 which are in fluid communication with nozzles 9. The air blower 5 generates pressurized air to a pressure greater than atmospheric pressure which is then fed through the primary hose 10, through the secondary hoses 11, and through the air pressure regulators 8 until it reaches the nozzles 9. In the example of FIG. 2, the air blower 5 generates air pressure by increasing the amount of air within the air delivery system. In some examples, the air blower 5 pressurizes the air in the primary hose 10 and hoses 11. In some examples, individual air blowers can be used to pressurize the air for individual nozzles while in other examples, a single air blower is used to pressurize the air for all the nozzles. The pressurized air is used by the nozzles 9 to generate a fog, mist, or spray of the treatment fluid by working in conjunction with a fluid delivery system 22 of FIG. 2 which is further described herein. In some examples, the air delivery system 21 can include an electric motor to generate the pressure for the treatment fluid such as an electric air blower. In some examples, pressurized air is provided by the exhaust from the primary vacuum of the surface maintenance machine or pressurized air is provided by the exhaust from the secondary vacuum of the surface maintenance machine. In some examples, pressurized air is provided by an electric air compressor. One having ordinary skill will appreciate other methods of generating pressurized air are contemplated.

As air blower 5 pressurizes the air in a primary hose 10 and hoses 11, the pressurized air feeds into individual air pressure regulators 8 through hoses 11. The air pressure regulators 8 can adjust the pressure of air delivered to each nozzle 9 which can be different for each individual nozzle 9. Being able to individually regulate the pressure of each nozzle 9 provides control over how far the fog, mist, or spray will reach for each nozzle 9. By combining and controlling the fog generated by each nozzle 9, an overall fog can be created which can consistently cover the desired area or surface. In some examples, only one air pressure regulator is used to control the pressure of air delivered to the nozzles such that the air pressure is the same for each nozzle. In other examples, no air pressure regulators are used, which can reduce the complexity of the system.

Continuing with FIG. 2, a fluid delivery system 22 includes a fluid pump 6, a fluid reservoir 4, and a fluid manifold 7 which is in fluid communication with nozzles 9 via hoses 15. In some examples, fluid pump 6 can be an electric motor. The fluid pump 6 pumps a fluid (e.g., a treatment fluid) from the fluid reservoir 4, which contains the fluid, through a hose 13 which can be a fluid hose. In some examples, fluid reservoir 4 can be a fluid reservoir of a surface maintenance machine (e.g., 120 in FIG. 1B. In some examples, the fluid reservoir 4 can be integrated as part of the fluid dispensing system 200. In some examples, the fluid reservoir 4 includes both a concentrated fluid (e.g., a concentrated treatment fluid) and a diluent (e.g., water) that mixes with the concentrated fluid before the combined fluids are dispensed. The fluid reservoir 4 may store the concentrated fluid and the diluent may be stored in separate containers or in separate portions of a single container. In some examples, the diluent may be used selectively with a concentrated fluid used in the fluid delivery system and selectively with a concentrated fluid (e.g., a cleaning fluid) used by the surface maintenance head assembly to clean the underlying floor on which the surface maintenance machine is traveling. That is, the fluid reservoir 4 could separately store a concentrated treatment fluid, a concentrated cleaning fluid, and a diluent (e.g., a larger supply than either of the concentrated fluids) that are selectively mixed and dispensed as appropriate. When activated, the fluid pump 6 pushes the fluid through a hose 14 connected to the fluid manifold 7. Fluid manifold 7 may distribute the fluid evenly to each of the nozzles 9; although in some designs the nozzles may be different such that the fluid is distributed unevenly. After the fluid has been distributed to the nozzles 9, the air delivery system 21 uses air pressure to force the fluid out of the nozzles in a fog, mist, or spray. Other methods can be used to deliver fluid to the nozzles 9. In some examples, the pressure differential created by the pressurized air of the air delivery system 21 could directly draw the fluid from the fluid reservoir 4 into the nozzle. In other examples, the fluid is delivered to the nozzles 9 by gravity. In still other examples, the fluid is delivered to the nozzles 9 by a siphoning force.

In some examples, a fluid pump pressurizes the fluid to a pressure greater than atmospheric pressure, such that the fluid delivery system 22 is able to dispense the fluid in a fog, mist, or spray. In this case, the fluid pump can be fluid pump 6, or it can be a separate fluid pump used for pressurizing the fluid. By pressurizing the fluid, the fluid can be dispensed through the one or more nozzles 9 without the need for an air delivery system 21. However, in some examples, the fluid delivery system 22 pressurizes the fluid and the air delivery system 21 pressurizes air. In such examples, by pressurizing both the air and the fluid, the fluid being dispensed by the nozzles in a fog, mist, or spray can cover a surface consistently.

In FIG. 2, the air delivery system 21 can be considered a pressurizing system as the air delivery system 21 pressurizes air to allow the fluid to be dispensed in a fog, mist, or spray. In some examples, the fluid delivery system 22 can be considered a pressurizing system as the fluid pump pressurizes the fluid to allow the treatment fluid to be dispensed in a fog, mist, or spray. In some examples, the air delivery system combined with the fluid delivery system can be considered a pressurizing system as they can pressurize air and the fluid to allow the fluid to be dispensed in a fog, mist, spray.

Further in the example of FIG. 2, a controller 3 is used to regulate the electric motors of the air delivery system 21 and fluid delivery system 22. In some examples, controller 3 can regulate the air pressure generated by the air delivery system 21 and/or the flow rate of the fluid of the fluid delivery system 22. In other examples, controller 3 can regulate the fluid pressure generated by the fluid pump. In some examples, the controller can also control a sequence of events regarding the air delivery system 21 and the fluid delivery system 22. In one example, the controller 3 builds enough air pressure in the air delivery system 21 before starting to provide fluid to the nozzles using the fluid delivery system 22.

In some aspects of the design, the fluid dispensing system 200 described herein relative to FIG. 2 may serve as a system separate from a surface maintenance machine (e.g., the surface maintenance machine 100 of FIG 1A) and is configured to be connected to such a surface maintenance machine, for instance, as a retrofit operation.

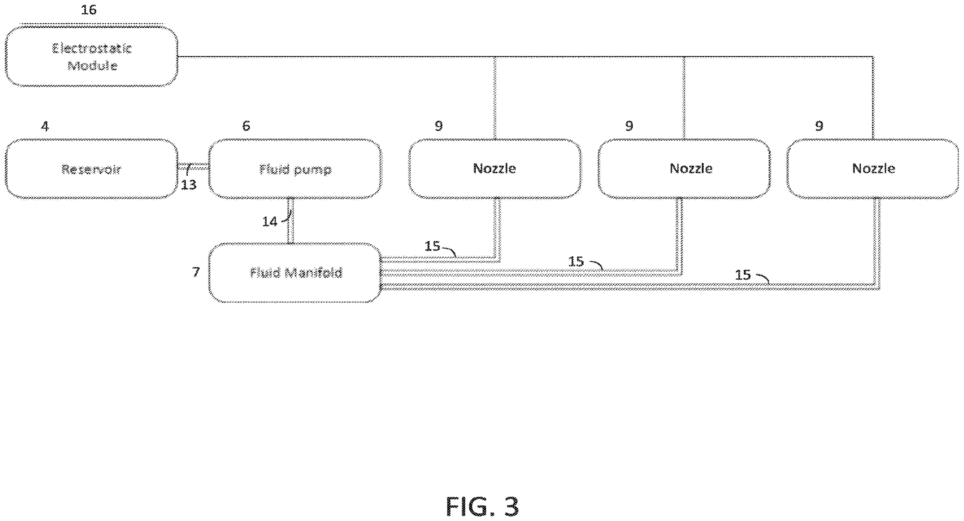

Moving to FIG. 3, FIG. 3 is a schematic view of an alternate embodiment of a fluid dispensing system including an electrostatic module 16. The electrostatic module 16 is connected to nozzles 9. Nozzles 9 are fluidly connected to a reservoir 4 through hoses 15, fluid manifold 7, hose 14, fluid pump 6, and finally hose 13. The electrostatic module 16 can apply an electrical charge to the fluid passing through it before the fluid is dispensed out of nozzles 9, thereby dispensing charged droplets of fluid. The charged droplets are attracted to surfaces, which can result in treatment fluids depositing faster on surfaces compared to non-charged droplets which tend to remain airborne for a longer duration before depositing on surfaces. In some examples, the charged droplets can wrap around the sides of a surface, thereby covering the backside of an object. In some examples, the electrostatic module 16 can be connected to the fluid delivery system of FIG. 2. In some aspects of the design, the fluid dispensing system described herein relative to FIG. 3 may serve as a system separate from a surface maintenance machine (e.g., the surface maintenance machine 100 of FIG 1A) and is configured to be connected to such a surface maintenance machine, for instance, as a retrofit operation.

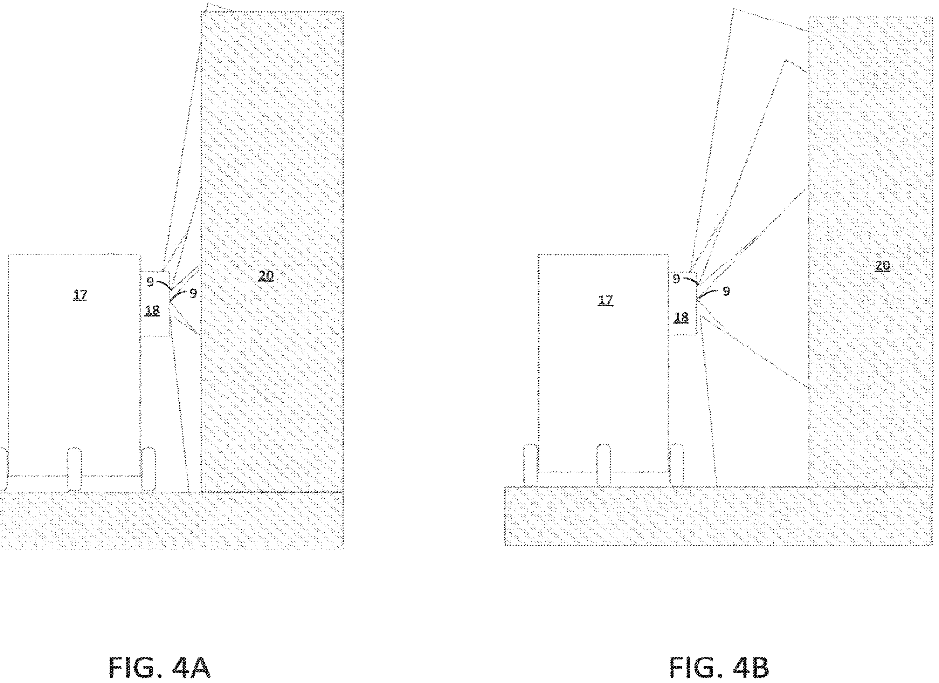

Moving to FIG. 4A and FIG. 4B, FIG. 4A and FIG. 4B are front elevation views of an example system including a fluid dispensing system 18 connected to a surface maintenance machine. In FIG. 4A, the fluid dispensing system 18 includes multiple nozzles 9 which dispense fluids such as a treatment fluid. The nozzles 9 provide coverage of a surface 20, which is a vertical surface, when the surface maintenance machine 17 is relatively close to the vertical surface. In some examples, the nozzles can dispense fluid on a portion of a surface outside of a maintenance operation zone (e.g., 126 in FIG. 1C). In some examples, the portion of the surface can be rightward or leftward relative to the surface maintenance machine as defined relative the forward direction of travel, including beyond the lateral extent of the maintenance operation zone. In some examples, the nozzles may dispense fluid in a rearward direction, as defined relative to the forward direction of travel of the surface maintenance machine, such that fluid is dispensed, in some examples, rearward of the maintenance operation zone (126 of FIG. 1C) or, in some examples, rearward of the extent of the body (132 of FIG. 1C) of the surface maintenance machine. In still further examples, the portion of the surface can be any combination of rightward, leftward, and/or rearward relative to the surface maintenance machine as defined relative to the forward direction of travel. In FIG. 4B, the surface maintenance machine 17 is further away when compared to FIG. 4A, however, it still provides coverage of the surface 20. The coverage of the surface 20 can be controlled by the nozzles 9. In the example of FIG. 4A and FIG. 4B, the nozzles 9 are directed at different angles relative to the horizontal plane and placed at different points on the fluid dispensing system 18. Additionally, in some embodiments, fog, mist, or spray can come out of the nozzles 9 at an angle (e.g., spray angle) separate from the angle of the nozzle. The angle and placement of the nozzles 9 along with the spray angle can allow the nozzles to fog, mist, or spray fluid toward the surface 20 in a controlled way to better cover the surface 20. With various configurations of angles, placement, and air pressure provided to the nozzles 9, the nozzles can be controlled to provide a consistent coverage of the surface from a top of the surface 20 to a bottom of the surface 20 with treatment fluid. In such configurations, the surface 20 would not have a portion which is not covered by some treatment fluid. In some examples, the fog, mist, or spray of fluid from the nozzles can be in a conical spray pattern or a flat spray pattern. In some embodiments the nozzles 9 configuration can be fixed, and in other embodiments, the angle and direction of the nozzles as well as the spray angle can be configurable.

In some examples, the portion of the surface 20 that is covered with the fluid (e.g., treatment fluid) can measure between 0.5 meters to 1.0 meters wide and between 0.0 meters (e.g., the floor surface) and 2.5 meters high. The portion that is covered can be considered the surface area that is accessible to a human. In which case, the surfaces that a human would touch are covered in treatment fluid and in some examples, are sanitized.

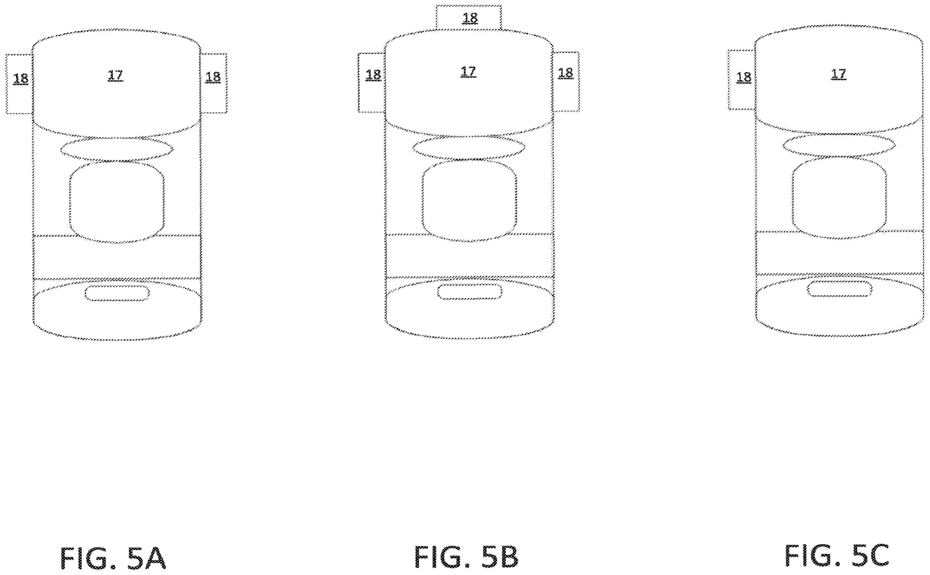

FIG. 5A to FIG. 5C show a top-down view of a surface maintenance machine 17 and placement of one or more nozzles in groups on the side of the surface maintenance machine 17. The groups of nozzles 18 can include one or more nozzles in various configurations. In FIG. 5A, the surface maintenance machine 17 is coupled to two groups of nozzles 18. One of the groups of nozzles is located on a first side of the surface maintenance machine 17 while the other group of nozzles is located on a second side, opposite the first side. This configuration can be used for covering surfaces in an environment where the corridor is relatively narrow. In some examples, the configuration of FIG. 5A is used for areas which extend up to two times the width of the surface maintenance machine 17. In FIG. 5B, the surface maintenance machine 17 further includes a third group of nozzles 18 located on a back side of the surface maintenance machine 17. The third group of nozzles 18 is coupled on the back of the surface maintenance machine and in some examples, can be pointed downward to a floor surface. In this configuration, the third group of nozzles can cover the floor surface with a treatment fluid. In FIG. 5C, only one group of nozzles 18 is coupled to the surface maintenance machine 17. In this configuration, the machine can clean an environment that is relatively large and open. In some examples, the configuration of FIG. 5C is used for areas which extend greater than two times the width of the surface maintenance machine 17. In FIG. 5A to FIG. 5C, the groups of nozzles 18 are all located behind a seat for the driver of the surface maintenance machine 17. In this configuration, a user in the seat and operating the surface maintenance machine 17 is protected from being covered by the treatment fluid dispensed from the group of nozzles 18. It will be appreciated that other configurations of fluid dispensing systems coupled to a surface maintenance machine are contemplated including varying the number and location of the fluid dispensing systems.

The various configurations of the groups of nozzles 18 on the surface maintenance machine 17 can allow a user to cover surfaces in a treatment fluid quickly and in some examples, the surfaces can be covered in a single pass of the surface maintenance machine 17 with the groups of nozzles 18. come out of the nozzles 9 at an angle (e.g., spray angle) separate from the angle of the nozzle. The angle and placement of the nozzles 9 along with the spray angle can allow the nozzles to fog, mist, or spray fluid toward the surface 20 in a controlled way to better cover the surface 20. With various configurations of angles, placement, and air pressure provided to the nozzles 9, the nozzles can be controlled to provide a consistent coverage of the surface from a top of the surface 20 to a bottom of the surface 20 with treatment fluid. In such configurations, the surface 20 would not have a portion which is not covered by some treatment fluid. In some examples, the fog, mist, or spray of fluid from the nozzles can be in a conical spray pattern or a flat spray pattern. In some embodiments the nozzles 9 configuration can be fixed, and in other embodiments, the angle and direction of the nozzles as well as the spray angle can be configurable.

In some examples, the portion of the surface 20 that is covered with the fluid (e.g., treatment fluid) can measure between 0.5 meters to 1.0 meters wide and between 0.0 meters (e.g., the floor surface) and 2.5 meters high. The portion that is covered can be considered the surface area that is accessible to a human. In which case, the surfaces that a human would touch are covered in treatment fluid and in some examples, are sanitized.

FIG. 5A to FIG. 5C show a top-down view of a surface maintenance machine 17 and placement of one or more nozzles in groups on the side of the surface maintenance machine 17. The groups of nozzles 18 can include one or more nozzles in various configurations. In FIG. 5A, the surface maintenance machine 17 is coupled to two groups of nozzles 18. One of the groups of nozzles is located on a first side of the surface maintenance machine 17 while the other group of nozzles is located on a second side, opposite the first side. This configuration can be used for covering surfaces in an environment where the corridor is relatively narrow. In some examples, the configuration of FIG. 5A is used for areas which extend up to two times the width of the surface maintenance machine 17. In FIG. 5B, the surface maintenance machine 17 further includes a third group of nozzles 18 located on a back side of the surface maintenance machine 17. The third group of nozzles 18 is coupled on the back of the surface maintenance machine and in some examples, can be pointed downward to a floor surface. In this configuration, the third group of nozzles can cover the floor surface with a treatment fluid. In FIG. 5C, only one group of nozzles 18 is coupled to the surface maintenance machine 17. In this configuration, the machine can clean an environment that is relatively large and open. In some examples, the configuration of FIG. 5C is used for areas which extend greater than two times the width of the surface maintenance machine 17. In FIG. 5A to FIG. 5C, the groups of nozzles 18 are all located behind a seat for the driver of the surface maintenance machine 17. In this configuration, a user in the seat and operating the surface maintenance machine 17 is protected from being covered by the treatment fluid dispensed from the group of nozzles 18. It will be appreciated that other configurations of fluid dispensing systems coupled to a surface maintenance machine are contemplated including varying the number and location of the fluid dispensing systems.

The various configurations of the groups of nozzles 18 on the surface maintenance machine 17 can allow a user to cover surfaces in a treatment fluid quickly and in some examples, the surfaces can be covered in a single pass of the surface maintenance machine 17 with the groups of nozzles 18.

## Claims

1. A surface maintenance machine (100) comprising:
a body (102) having an extent;
wheels (103) for supporting the body (102) over a first surface for movement in a forward direction of travel (208);
surface maintenance equipment (136) supported by the body (102) and extending towards the first surface configured to perform a surface maintenance operation on at least a portion of the first surface defined as a maintenance operation zone (126), the surface maintenance equipment including a maintenance head assembly (116) comprising one or more motor-driven surface maintenance tools (118) for performing the surface maintenance operation within the maintenance operation zone (126);
a treatment fluid reservoir (4) adapted to store a treatment fluid and carried by the body (102) of the surface maintenance machine (100);
a pressurizing system (21) adapted to pressurize at least one of the treatment fluid and an air pressure to a pressure greater than atmospheric pressure; and
one or more nozzles (9) in fluid communication with the treatment fluid reservoir (4) and the pressurizing system (21), the one or more nozzles (9) configured to receive the treatment fluid, **characterized in that** the one or more nozzles (9) are configured to dispense the treatment fluid to a second surface, the one or more nozzles configured to be located and oriented to dispense the treatment fluid to the second surface in at least one of the direction of:
a. rightward of the surface maintenance machine as defined relative to the forward direction of travel (208), including rightward of and beyond the extent of the body,
b. leftward of the surface maintenance machine as defined relative to the forward direction of travel (208), including leftward of and beyond the extent of the body, and
c. rearward of the surface maintenance machine as defined relative to the forward direction of travel (208), including rearward of and beyond the extent of the body.

2. The surface maintenance machine (100) of claim 1, wherein the surface maintenance equipment (136) includes a vacuum supported by the body (102), the vacuum for withdrawing substances from the first surface along a lateral extent (128) of the maintenance operation zone (126).

3. The surface maintenance machine (100) of claim 2 or any of the preceding claims, further including a cleaning fluid reservoir (120) carried by the body (102), the cleaning fluid reservoir (120) configured to provide a cleaning fluid to the first surface within the maintenance operation zone (126), and wherein the vacuum is a vacuum squeegee (124b) for withdrawing fluid from the first surface along the lateral extent (128) of the maintenance operation zone (126).

4. The surface maintenance machine (100) of claim 3 or any of the preceding claims,
wherein the treatment fluid reservoir (4) and the cleaning fluid reservoir (120) are the same reservoir, and wherein the reservoir is fluidly connected to the maintenance head assembly (116) and to the one or more nozzles (9).

5. The surface maintenance machine (100) of the claim 1 or any of the preceding claims, further comprising an air blower (5), the air blower configured to pressurize air to the pressure greater than atmospheric pressure, the pressurized air being used to dispense the treatment fluid to the second surface using the one or more nozzles (9).

6. The surface maintenance machine (100) of claim 5 or any of the preceding claims, further comprising one or more air pressure regulators (8) in fluid communication with the one or more nozzles (9) and the air blower (5), the one or more air pressure regulators (8) configured to change the air pressure of each the one or more nozzles (9) independently of each other.

7. The surface maintenance machine (100) of claim 1 or any of the preceding claims,
wherein at least one of the one or more nozzles (9) is located on a first side of the surface maintenance machine and a second side of the surface maintenance machine, each of the one or more nozzles (9) being located reward of a seat of the surface maintenance machine (100) as defined relative to the forward direction of travel (208).

8. The surface maintenance machine (100) of claim 1 or any of the preceding claims, wherein the second surface is located vertically above the first surface, the first surface being below the surface maintenance machine (100).

9. The surface maintenance machine (100) of claim 1 or any of the preceding claims, further comprising one or more electrostatic modules (16) located at the one or more nozzles (9) configured to electrically charge the treatment fluid.

10. The surface maintenance machine (100) of claim 1 or any of the preceding claims, wherein the pressurizing system (21) includes an air pressurizing system provided by an electric air compressor.

11. The surface maintenance machine (100) of claim 1 or any of the preceding claims, further comprising a pump (6) configured to deliver the treatment fluid from the treatment fluid reservoir (4) to a manifold (7), the manifold dispensing the treatment fluid to the one or more nozzles (9).

12. A method for dispensing fluid comprising:
performing a surface maintenance operation on a portion of a first surface, defined as a maintenance operation zone (126), using a surface maintenance machine (100), the surface maintenance machine comprising:
a body (102);
wheels (103) for supporting the body (102) over the first surface for movement in a forward direction of travel (208); and
a maintenance head assembly (116) comprising one or more motor-driven surface maintenance tools (118) extending toward the first surface for performing the surface maintenance operation within the maintenance operation zone (126);
dispensing a treatment fluid on a second surface using a treatment fluid dispensing system while performing the surface maintenance operation, the treatment fluid dispensing system comprising:
a treatment fluid reservoir (4) adapted to store the treatment fluid;
a pressurizing system (21) adapted to pressurize at least one of the treatment fluid and an air pressure to a pressure greater than atmospheric pressure; and
one or more nozzles (9) in fluid communication with the treatment fluid reservoir (4) and the pressurizing system (21) such that the one or more nozzles (9) receive the treatment fluid and dispense the treatment fluid, the one or more nozzles being located and oriented to dispense the treatment fluid on the second surface, the treatment fluid treating the second surface; and
moving the surface maintenance machine (100) over the first surface in the forward direction of travel (208).

13. The method of claim 12 or any of the preceding claims, wherein the moving the surface maintenance machine (100) over the first surface in the forward direction of travel (208) occurs while dispensing the treatment fluid on the second surface.

14. The method of claim 12 or any of the preceding claims, where the first surface, the maintenance operation zone (126), and the second surface change as the surface maintenance machine moves over the first surface in the forward direction of travel (208).

15. The method according to claim 12 or any of the preceding claims, wherein the dispensing the treatment fluid on the second surface includes each of the one or more nozzles (9) dispensing the treatment fluid such that the second surface is covered by the treatment fluid when the one or more nozzles are up to 2.0 meters away from the second surface.

## Patentansprüche

1. Wartungsmaschine (100) für Oberflächen, umfassend:
einen Körper (102), der eine Ausdehnung aufweist;
Räder (103) zum Tragen des Körpers (102) über einer ersten Oberfläche für eine Bewegung in einer Vorwärtsfahrtrichtung (208);
eine Oberflächenwartungsausrüstung (136), die durch den Körper (102) getragen wird und sich in Richtung der ersten Oberfläche erstreckt, die konfiguriert ist, um einen Oberflächenwartungsvorgang auf mindestens einem Abschnitt der ersten Oberfläche durchzuführen, der als eine Wartungsvorgangszone (126) definiert ist, die Oberflächenwartungsausrüstung einschließlich einer Wartungskopfanordnung (116), umfassend ein oder mehrere motorbetriebene Oberflächenwartungswerkzeuge (118) zum Durchführen des Oberflächenwartungsvorgangs innerhalb der Wartungsvorgangszone (126);
einen Behandlungsfluidbehälter (4), der angepasst ist, um ein Behandlungsfluid aufzubewahren, und durch den Körper (102) der Oberflächenwartungsmaschine (100) gestützt wird;
ein Drucksystem (21), das angepasst ist, um mindestens eines des Behandlungsfluids und eines Luftdrucks auf einen Druck unter Druck zu setzen, der höher als der Atmosphärendruck ist; und
eine oder mehrere Düsen (9) in Fluidverbindung mit dem Behandlungsfluidbehälter (4) und dem Drucksystem (21), wobei die eine oder die mehreren Düsen (9) konfiguriert sind, um das Behandlungsfluid aufzunehmen, **dadurch gekennzeichnet, dass** die eine oder die mehreren Düsen (9) konfiguriert sind, um das Behandlungsfluid auf eine zweite Oberfläche abzugeben, wobei die eine oder die mehreren Düsen konfiguriert sind, um gelegen und ausgerichtet zu sein, um das Behandlungsfluid auf die zweite Oberfläche in mindestens eine der Richtung abzugeben von:
a. rechts von der Oberflächenwartungsmaschine, wie definiert relativ zu der Vorwärtsfahrtrichtung (208), einschließlich rechts von und über die Ausdehnung des Körpers hinaus,
b. links von der Oberflächenwartungsmaschine, wie definiert relativ zu der Vorwärtsfahrtrichtung (208), einschließlich links von und über die Ausdehnung des Körpers hinaus, und
c. hinter der Oberflächenwartungsmaschine, wie definiert in Bezug auf die Vorwärtsfahrtrichtung (208), einschließlich hinter und über die Ausdehnung des Körpers hinaus.

2. Oberflächenwartungsmaschine (100) nach Anspruch 1, wobei die Oberflächenwartungsausrüstung (136) ein Vakuum einschließt, das durch den Körper (102) getragen wird, wobei das Vakuum zum Absaugen von Substanzen von der ersten Oberfläche entlang einer seitlichen Ausdehnung (128) der Wartungsbetriebszone (126) dient.

3. Oberflächenwartungsmaschine (100) nach Anspruch 2 oder einem der vorstehenden Ansprüche, ferner einschließlich eines Reinigungsfluidbehälters (120), der durch den Körper (102) gestützt wird, wobei der Reinigungsfluidbehälter (120) konfiguriert ist, um ein Reinigungsfluid der ersten Oberfläche innerhalb der Wartungsbetriebszone (126) bereitzustellen, und wobei das Vakuum ein Vakuumabzieher (124b) zum Absaugen von Fluid von der ersten Oberfläche entlang der seitlichen Ausdehnung (128) der Wartungsbetriebszone (126) ist.

4. Oberflächenwartungsmaschine (100) nach Anspruch 3 oder einem der vorstehenden Ansprüche, wobei der Behandlungsfluidbehälter (4) und der Reinigungsfluidbehälter (120) derselbe Behälter sind und wobei der Behälter mit der Wartungskopfanordnung (116) und mit der einen oder den mehreren Düsen (9) fluidisch verbunden ist.

5. Oberflächenwartungsmaschine (100) nach Anspruch 1 oder einem der vorstehenden Ansprüche, ferner umfassend ein Luftgebläse (5), wobei das Luftgebläse konfiguriert ist, um Luft auf den Druck unter Druck zu setzen, der höher als der Atmosphärendruck ist, wobei die Druckluft verwendet wird, um das Behandlungsfluid unter Verwendung der einen oder der mehreren Düsen (9) auf die zweite Oberfläche abzugeben.

6. Oberflächenwartungsmaschine (100) nach Anspruch 5 oder einem der vorstehenden Ansprüche, ferner umfassend einen oder mehrere Luftdruckregler (8) in Fluidverbindung mit der einen oder den mehreren Düsen (9) und dem Luftgebläse (5), wobei der eine oder die mehreren Luftdruckregler (8) konfiguriert sind, um den Luftdruck jeder der einen oder der mehreren Düsen (9) unabhängig voneinander zu ändern.

7. Oberflächenwartungsmaschine (100) nach Anspruch 1 oder einem der vorstehenden Ansprüche, wobei mindestens eine der einen oder der mehreren Düsen (9) auf einer ersten Seite der Oberflächenwartungsmaschine und einer zweiten Seite der Oberflächenwartungsmaschine gelegen ist, wobei jede der einen oder der mehreren Düsen (9) wie definiert relativ zu der Vorwärtsfahrtrichtung (208) hinter einem Sitz der Oberflächenwartungsmaschine (100) gelegen ist.

8. Oberflächenwartungsmaschine (100) nach Anspruch 1 oder einem der vorstehenden Ansprüche, wobei die zweite Oberfläche vertikal über der ersten Oberfläche gelegen ist und die erste Oberfläche unterhalb der Oberflächenwartungsmaschine (100) liegt.

9. Oberflächenwartungsmaschine (100) nach Anspruch 1 oder einem der vorstehenden Ansprüche, ferner umfassend ein oder mehrere elektrostatische Module (16), die an der einen oder den mehreren Düsen (9) gelegen sind, die konfiguriert sind, um das Behandlungsfluid elektrisch aufzuladen.

10. Oberflächenwartungsmaschine (100) nach Anspruch 1 oder einem der vorstehenden Ansprüche, wobei das Drucksystem (21) ein Luftdrucksystem einschließt, das durch einen elektrischen Luftkompressor bereitgestellt wird.

11. Oberflächenwartungsmaschine (100) nach Anspruch 1 oder einem der vorstehenden Ansprüche, ferner umfassend eine Pumpe (6), die konfiguriert ist, um das Behandlungsfluid aus dem Behandlungsfluidbehälter (4) zu einem Verteiler (7) zu fördern, wobei der Verteiler das Behandlungsfluid an die eine oder die mehreren Düsen (9) abgibt.

12. Verfahren zum Abgeben von Fluid, umfassend:
Durchführen eines Oberflächenwartungsvorgangs auf einem Abschnitt einer ersten Oberfläche, der als eine Wartungsvorgangszone (126) definiert ist, unter Verwendung einer Oberflächenwartungsmaschine (100), die Oberflächenwartungsmaschine umfassend:
einen Körper (102);
Räder (103) zum Tragen des Körpers (102) über der ersten Oberfläche für die Bewegung in einer Vorwärtsfahrtrichtung (208); und
eine Wartungskopfanordnung (116), umfassend ein oder mehrere motorbetriebene Oberflächenwartungswerkzeuge (118), die sich in Richtung der ersten Oberfläche erstrecken, zum Durchführen des Oberflächenwartungsvorgangs innerhalb der Wartungsvorgangszone (126);
Abgeben eines Behandlungsfluids auf eine zweite Oberfläche unter Verwendung eines Behandlungsfluidabgabesystems während der Oberflächenwartungsvorgang durchgeführt wird, das Behandlungsfluidabgabesystem umfassend:
einen Behandlungsfluidbehälter (4), der angepasst ist, um das Behandlungsfluid aufzubewahren;
ein Drucksystem (21), das angepasst ist, um mindestens eines des Behandlungsfluids und eines Luftdrucks auf einen Druck unter Druck zu setzen, der höher als der Atmosphärendruck ist; und
eine oder mehrere Düsen (9) in Fluidverbindung mit dem Behandlungsfluidbehälter (4) und dem Drucksystem (21), sodass die eine oder die mehreren Düsen (9) das Behandlungsfluid aufnehmen und abgeben, wobei die eine oder die mehreren Düsen gelegen und ausgerichtet sind, um das Behandlungsfluid auf die zweite Oberfläche abzugeben, wobei das Behandlungsfluid die zweite Oberfläche behandelt; und
Bewegen der Oberflächenwartungsmaschine (100) über die erste Oberfläche in der Vorwärtsfahrtrichtung (208).

13. Verfahren nach Anspruch 12 oder einem der vorstehenden Ansprüche, wobei das Bewegen der Oberflächenwartungsmaschine (100) über die erste Oberfläche in der Vorwärtsfahrtrichtung (208) erfolgt, während das Behandlungsfluid auf die zweite Oberfläche abgegeben wird.

14. Verfahren nach Anspruch 12 oder einem der vorstehenden Ansprüche, wobei sich die erste Oberfläche, die Wartungsbetriebszone (126) und die zweite Oberfläche ändern, wenn sich die Oberflächenwartungsmaschine in der Vorwärtsfahrtrichtung (208) über die erste Oberfläche bewegt.

15. Verfahren nach Anspruch 12 oder einem der vorstehenden Ansprüche, wobei das Abgeben des Behandlungsfluids auf die zweite Oberfläche das Abgeben der Behandlungsfluid durch jede der einen oder der mehreren Düsen (9) so einschließt, dass die zweite Oberfläche durch das Behandlungsfluid bedeckt ist, wenn die eine oder die mehreren Düsen bis zu 2,0 Meter von der zweiten Oberfläche entfernt sind.

## Revendications

1. Machine d'entretien de surface (100) comprenant :
un corps (102) présentant une étendue ;
des roues (103) pour supporter le corps (102) sur une première surface afin de le déplacer dans une direction de déplacement vers l'avant (208) ;
un équipement d'entretien de surface (136) supporté par le corps (102) et s'étendant vers la première surface conçu pour effectuer une opération d'entretien de surface sur au moins une partie de la première surface définie comme zone d'opération d'entretien (126), l'équipement d'entretien de surface comportant un ensemble de tête d'entretien (116) comprenant un ou plusieurs outils d'entretien de surface motorisés (118) pour effectuer l'opération d'entretien de surface à l'intérieur de la zone d'opération d'entretien (126) ;
un réservoir de fluide de traitement (4) adapté pour stocker un fluide de traitement et porté par le corps (102) de la machine d'entretien de surface (100) ;
un système de pressurisation (21) adapté pour pressuriser au moins l'un parmi le fluide de traitement et une pression d'air à une pression supérieure à la pression atmosphérique ; et
une ou plusieurs buses (9) en communication fluidique avec le réservoir de fluide de traitement (4) et le système de pressurisation (21), la ou les buses (9) conçues pour recevoir le fluide de traitement, **caractérisée en ce que** la ou les buses (9) sont conçues pour distribuer le fluide de traitement sur une seconde surface, la ou les
buses conçues pour être situées et orientées de manière à distribuer le fluide de traitement sur la seconde surface dans au moins l'une des directions suivantes :
a. vers la droite de la machine d'entretien de surface telle que définie par rapport à la direction de déplacement vers l'avant (208), y compris vers la droite et au-delà de l'étendue du corps,
b. vers la gauche de la machine d'entretien de surface telle que définie par rapport à la direction de déplacement vers l'avant (208), y compris vers la gauche et au-delà de l'étendue du corps, et
c. à l'arrière de la machine d'entretien de surface telle que définie par rapport à la direction de déplacement vers l'avant (208), y compris à l'arrière et au-delà de l'étendue du corps.

2. Machine d'entretien de surface (100) selon la revendication 1, dans laquelle l'équipement d'entretien de surface (136) comporte un aspirateur supporté par le corps (102), l'aspirateur permettant de retirer des substances de la première surface le long d'une étendue latérale (128) de la zone d'opération d'entretien (126).

3. Machine d'entretien de surface (100) selon la revendication 2 ou l'une quelconque des revendications précédentes, comportant en outre un réservoir de fluide de nettoyage (120) porté par le corps (102), le réservoir de fluide de nettoyage (120) conçu pour fournir un fluide de nettoyage à la première surface à l'intérieur de la zone d'opération d'entretien (126), et dans laquelle l'aspirateur est une raclette aspirante (124b) pour retirer du fluide de la première surface le long de l'étendue latérale (128) de la zone d'opération d'entretien (126).

4. Machine d'entretien de surface (100) selon la revendication 3 ou l'une quelconque des revendications précédentes, dans laquelle le réservoir de fluide de traitement (4) et le réservoir de fluide de nettoyage (120) sont le même réservoir, et dans laquelle le réservoir est relié fluidiquement à l'ensemble de la tête d'entretien (116) et à une ou plusieurs buses (9).

5. Machine d'entretien de surface (100) selon la revendication 1 ou l'une quelconque des revendications précédentes, comprenant en outre un ventilateur (5), le ventilateur étant conçu pour pressuriser l'air à une pression supérieure à la pression atmosphérique, l'air pressurisé étant utilisé pour distribuer le fluide de traitement sur la seconde surface à l'aide d'une ou de plusieurs buses (9).

6. Machine d'entretien de surface (100) selon la revendication 5 ou l'une quelconque des revendications précédentes, comprenant en outre un ou plusieurs régulateurs de pression d'air (8) en communication fluidique avec la ou les buses (9) et le ventilateur (5), le ou les régulateurs de pression d'air (8) étant conçus pour modifier la pression d'air de chacune parmi la ou les buses (9) indépendamment l'une de l'autre.

7. Machine d'entretien de surface (100) selon la revendication 1 ou l'une quelconque des revendications précédentes, dans laquelle au moins une parmi la ou les buses (9) est située sur un premier côté de la machine d'entretien de surface et sur un second côté de la machine d'entretien de surface, chacune parmi la ou les buses (9) étant située à l'arrière d'un siège de la machine d'entretien de surface (100) tel que défini par rapport à la direction de déplacement vers l'avant (208).

8. Machine d'entretien de surface (100) selon la revendication 1 ou l'une quelconque des revendications précédentes, dans laquelle la seconde surface est située verticalement au-dessus de la première surface, la première surface étant en dessous de la machine d'entretien de surface (100).

9. Machine d'entretien de surface (100) selon la revendication 1 ou l'une quelconque des revendications précédentes, comprenant en outre un ou plusieurs modules électrostatiques (16) situés au niveau de la ou des buses (9) et conçus pour charger électriquement le fluide de traitement.

10. Machine d'entretien de surface (100) selon la revendication 1 ou l'une quelconque des revendications précédentes, dans laquelle le système de pressurisation (21) comporte un système de pressurisation d'air fourni par un compresseur d'air électrique.

11. Machine d'entretien de surface (100) selon la revendication 1 ou l'une quelconque des revendications précédentes, comprenant en outre une pompe (6) conçue pour acheminer le fluide de traitement du réservoir de fluide de traitement (4) vers un collecteur (7), le collecteur distribuant le fluide de traitement à la ou les buses (9).

12. Procédé de distribution de fluide comprenant :
le fait d'effectuer une opération d'entretien de surface sur une partie d'une première surface, définie comme une zone d'opération d'entretien (126), à l'aide d'une machine d'entretien de surface (100), la machine d'entretien de surface comprenant :
un corps (102) ;
des roues (103) pour supporter le corps (102) sur la première surface afin de le déplacer dans une direction de déplacement vers l'avant (208) ; et
un ensemble de tête d'entretien (116) comprenant un ou plusieurs outils d'entretien de surface motorisés (118) s'étendant vers la première surface pour effectuer l'opération d'entretien de surface dans la zone d'opération d'entretien (126) ;
la distribution d'un fluide de traitement sur une seconde surface à l'aide d'un système de distribution de fluide de traitement tout en effectuant l'opération d'entretien de la surface, le système de distribution de fluide de traitement comprenant :
un réservoir de fluide de traitement (4) adapté pour stocker le fluide de traitement ;
un système de pressurisation (21) adapté pour pressuriser au moins l'un parmi le fluide de traitement et une pression d'air à une pression supérieure à la pression atmosphérique ; et
une ou plusieurs buses (9) en communication fluidique avec le réservoir de fluide de traitement (4) et le système de pressurisation (21) de sorte que la ou les buses (9) reçoivent le fluide de traitement et distribuent le fluide de traitement, la ou les buses étant situées et orientées de manière à distribuer le fluide de traitement sur la seconde surface, le fluide de traitement traitant la seconde surface ; et
le déplacement de la machine d'entretien des surfaces (100) sur la première surface dans la direction de déplacement vers l'avant (208).

13. Procédé selon la revendication 12 ou l'une quelconque des revendications précédentes, dans lequel le déplacement de la machine d'entretien de surface (100) sur la première surface dans la direction de déplacement vers l'avant (208) se produit pendant la distribution du fluide de traitement sur la deuxième surface.

14. Procédé selon la revendication 12 ou l'une quelconque des revendications précédentes, où la première surface, la zone d'opération d'entretien (126), et la seconde surface changent au fur et à mesure que la machine d'entretien de surface se déplace sur la première surface dans la direction de déplacement vers l'avant (208).

15. Procédé selon la revendication 12 ou l'une quelconque des revendications précédentes, dans lequel la distribution du fluide de traitement sur la seconde surface comporte la distribution du fluide de traitement par chacune parmi la ou les buses (9) de manière à ce que la seconde surface soit couverte par le fluide de traitement lorsque la ou les buses se trouvent à une distance maximale de 2,0 mètres de la seconde surface.
